(19) Europäisches Patentamt
European Patent Office
Office européen des brevets

(11) Veröffentlichungsnummer : **0 019 190**
**B1**

(12) # EUROPÄISCHE PATENTSCHRIFT

(45) Veröffentlichungstag der Patentschrift : **30.03.83**

(51) Int. Cl.³ : **A 01 N 43/50**

(21) Anmeldenummer : **80102451.4**

(22) Anmeldetag : **06.05.80**

(54) **Verwendung von Hydroxyalkyl-imidazolen als Fungizide.**

(30) Priorität : **19.05.79 DE 2920375**

(43) Veröffentlichungstag der Anmeldung : **26.11.80 Patentblatt 80/24**

(45) Bekanntmachung des Hinweises auf die Patenterteilung : **30.03.83 Patentblatt 83/13**

(84) Benannte Vertragsstaaten : **BE CH DE FR GB IT LI NL**

(56) Entgegenhaltungen :
**EP A 0 005 250**
**FR A 2 399 803**

**Die Akte enthält technische Angaben, die nach dem Eingang der Anmeldung eingereicht wurden und die nicht in dieser Patentschrift enthalten sind.**

(73) Patentinhaber : **BAYER AG**
**Zentralbereich Patente, Marken und Lizenzen**
**D-5090 Leverkusen 1, Bayerwerk (DE)**

(72) Erfinder : **Regel, Erik, Ing-grad.**
**Bergerheide 72a**
**D-5600 Wuppertal 1 (DE)**
Erfinder : **Büchel, Karl-Heinz, Dr. Prof.**
**Bergerheide 62**
**D-5600 Wuppertal 1 (DE)**
Erfinder : **Brandes, Wilhelm, Dr.**
**Eichendorffstrasse 3**
**D-5653 Leichlingen (DE)**
Erfinder : **Paul, Volker, Dr.**
**Ahornstrasse 5**
**D-5650 Solingen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 019 190 B1

# Verwendung von Hydroxyalkyl-imidazolen als Fungizide

Die vorliegende Erfindung betrifft die Verwendung von Hydroxyalkyl -imidazolen als Fungizide zur Bekämpfung von pflanzenschädigenden Pilzen.

Es ist bereits bekannt geworden, daß substituierte 1-Phenyl-2-imidazolyl-1-ethanole, wie z.B. 1-Halogenphenyl-2-imidazolyl-1-ethanole, zur Herstellung von fungizid wirksamen 1-(β-Phenyl)-ethyl-imidazolethern verwendet werden können (vergleiche US-Patentschrift 3 717 655). Die fungizide Wirksamkeit dieser Zwischenprodukte ist jedoch, insbesondere bei niedrigen Aufwandmengen und -konzentrationen, nicht immer befriedigend. Weiterhin sind fungizid wirksame 2-Hydroxyalkyl-imidazol-Derivate bekannt (vergleiche Französische Patentschrift Nr. 2 399 802), deren Wirkung jedoch, zum Beispiel gegen Podosphaera-Arten, nicht immer voll befriedigt. Endlich sind noch Hydroxypropyl-imidazol-Derivate, deren Herstellung und Verwendung als Arzneimittel beschrieben (veröffentlichte Europäische Patentanmeldung Nr. 0 005 250).

Es wurde gefunden, daß die Hydroxyalkyl-imidazole der allgemeinen Formel

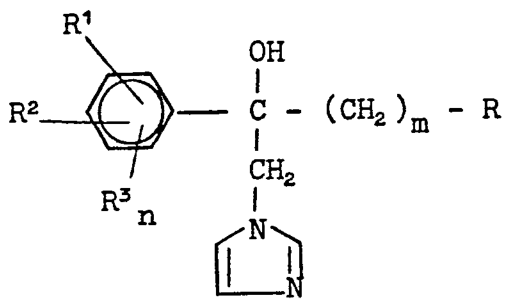

(I)

in welcher

R für gegebenenfalls durch Halogen oder Alkyl mit 1 bis 4 Kohlenstoffatomen substituiertes Phenyl, Naphthyl oder Tetrahydronaphthyl steht,

$R^1$ für gegebenenfalls durch Halogen, Alkyl oder Alkenyl mit jeweils bis zu 4 Kohlenstoffatomen substituiertes Phenyl oder Cycloalkyl mit 3 bis 7 Kohlenstoffatomen steht und

$R^2$ für Wasserstoff steht oder

$R^1$ und $R^2$ gemeinsam in o-Stellung zueinander für eine gegebenenfalls durch Halogen oder Alkyl mit bis zu 2 Kohlenstoffatomen substituierte Methylenbrücke mit 3 bis 5 Methylengruppen oder zusammen mit dem Phenylring für Naphthyl stehen,

$R^3$ für Halogen oder Alkyl mit 1 bis 4 Kohlenstoffatomen steht,

n für die Zahlen 0, 1 oder 2 steht und

m für die Zahlen 0 oder 1 steht,

und deren physiologisch verträglichen Säureadditions-Salze gute fungizide Eigenschaften zur Bekämpfung von pflanzenschädigenden Pilzen aufweisen.

Ueberraschenderweise zeigen die erfindungsgemäß verwendbaren Hydroxyalkyl-imidazole der Formel (I) eine bessere fungizide Wirksamkeit als die aus dem Stand der Technik bekannten 1-Halogenphenyl-2-imidazolyl-1-ethanole, welche chemisch naheliegende Verbindungen sind. Die erfindungsgemäß verwendbaren Stoffe stellen somit eine Bereicherung der Technik dar.

Bevorzugt sind diejenigen Verbindungen der Formel (I), in denen R für Phenyl, das gegebenenfalls einfach oder zweifach, gleich oder verschieden durch Chlor, Fluor oder Methyl substituiert ist, für Naphthyl und Tetrahydronaphthyl steht ; $R^1$ für Phenyl, Cyclopentyl oder Cyclohexyl steht, diese Reste können gegebenenfalls einfach oder zweifach durch Chlor, Brom, Fluor, Methyl, Ethyl, Isopropyl oder Isopropenyl substituiert sein ; und $R^2$ für Wasserstoff steht, oder $R^1$ und $R^2$ gemeinsam in ortho-Stellung zueinander für eine Tri-, Tetra- oder Pentamethylenbrücke, die gegebenenfalls durch Chlor oder Methyl substituiert ist, oder zusammen mit dem Phenylring für Naphthyl stehen ; $R^3$ für Chlor, Fluor oder Methyl steht, n für 0 oder 1 steht und m für 0 oder 1 steht.

Im Einzelnen seien außer den bei den Herstellungsbeispielen genannten Verbindungen die folgenden Verbindungen der allgemeinen Formel (I) genannt :

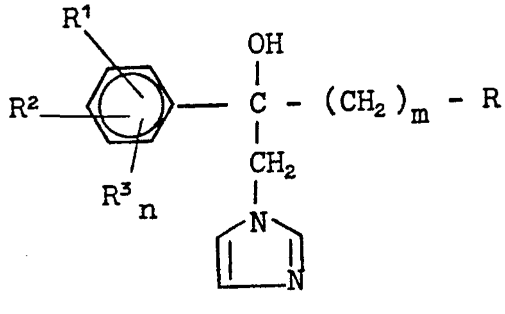

(I)

| R | $R^1$ | $R^2$ | $R^3_n$ | m |
|---|---|---|---|---|
| 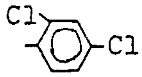 | 4-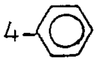 | H | - | 0 |
| 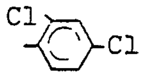 | 4-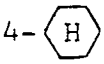 | H | - | 0 |
| 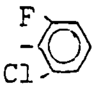 | 4-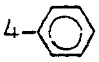 | H | - | 0 |
| 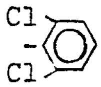 | 4-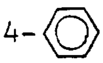 | H | - | 0 |
| 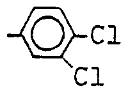 | 4-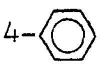 | H | - | 0 |
| 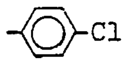 | $3,4\text{-}(CH_2)_3\text{-}$ | | - | 0 |
| 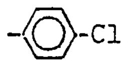 | $3,4\text{-}(CH_2)_4\text{-}$ | | - | 0 |
| 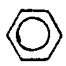 | 4-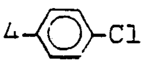 | H | - | 0 |
| 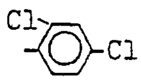 | 4-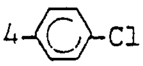 | H | - | 0 |
| 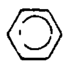 | 4-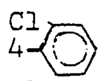 | H | - | 0 |
| 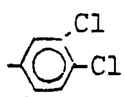 | 4-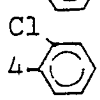 | H | - | 0 |
| 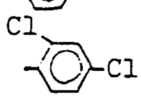 | 4-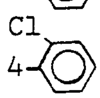 | H | - | 0 |
| 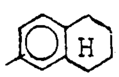 | 4-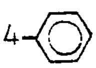 | H | - | 0 |
| 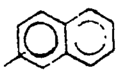 | 4-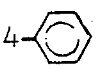 | H | - | 0 |
| 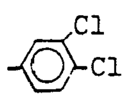 | 4-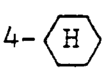 | H | - | 0 |
| 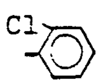 | $3,4\text{-}(CH_2)_4\text{-}$ | | - | 0 |
| 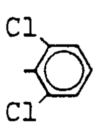 | $3,4\text{-}(CH_2)_3\text{-}$ | | - | 0 |
| 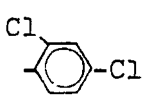 | 4-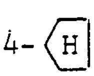 | H | - | 0 |
| 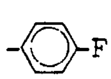 | 4-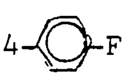 | H | - | 0 |
| 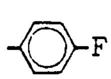 | 4-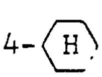 | H | - | 0 |

(Fortsetzung)

| R | $R^1$ | $R^2$ | $R^3_n$ | m |
|---|---|---|---|---|
| 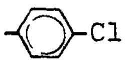 | 4-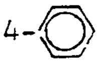 | H | - | 0 |
| 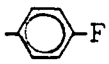 | 4-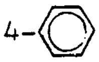 | H | - | 0 |
| 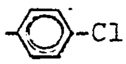 | 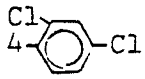 | H | - | 0 |
| 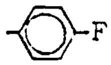 | 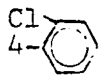 | H | - | 0 |
| 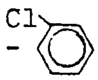 | 4-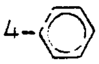 | H | - | 1 |
| 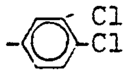 | 4-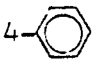 | H | - | 1 |
| 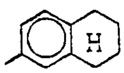 | 4-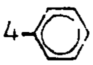 | H | - | 1 |
| 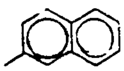 | 4-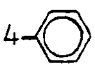 | H | - | 1 |
| 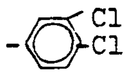 | 4-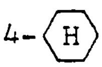 | H | - | 1 |
| 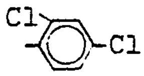 | 4-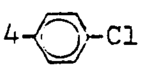 | H | - | 1 |
| 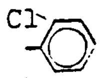 | $3,4\text{-}(CH_2)_4\text{-}$ | | - | 1 |
| 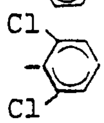 | $3,4\text{-}(CH_2)_3\text{-}$ | | - | 1 |
| 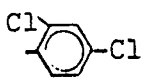 | 4-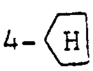 | H | - | 1 |
| 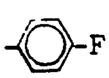 | 4-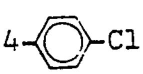 | H | - | 1 |
| 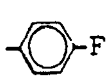 | 4-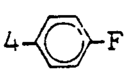 | H | - | 1 |
| 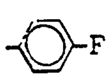 | 4-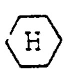 | H | - | 1 |
| 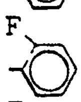 | 4-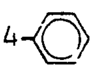 | H | - | 1 |
| 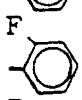 | 4-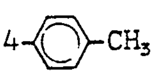 | H | - | 1 |
| 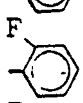 | 4-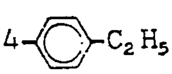 | H | - | 1 |
| 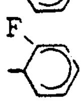 | 4-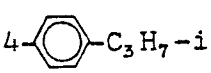 | H | - | 1 |

(Fortsetzung)

| R | $R^1$ | $R^2$ | $R^3_n$ | m |
|---|---|---|---|---|
| 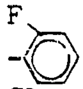 | 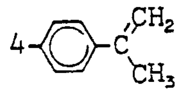 | H | - | 1 |
| 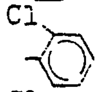 | 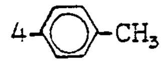 | H | - | 1 |
| 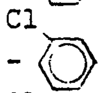 | 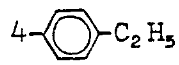 | H | - | 1 |
| 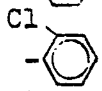 | 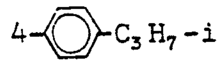 | H | - | 1 |
| 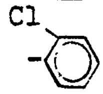 | 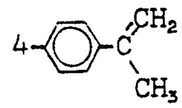 | H | - | 1 |

Die erfindungsgemäß zu verwendenden Wirkstoffe und deren Säureadditionssalze und deren Herstellung sind Gegenstand von eigenen älteren Anmeldungen (vergleiche die Deutschen Offenlegungsschriften D 2 820 489, D 28 32 677 und D 28 51 116) ; und können nach den dort beschriebenen Verfahren hergestellt werden, indem man

a) Imidazolylmethyl-phenyl-ketone der Formel

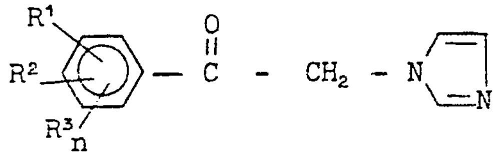

(II)

in welcher $R^1$, $R^2$, $R^3$ und n die oben angegebene Bedeutung haben, mit einer Grignard-Verbindung der Formel

$$R—(CH_2)_m—Mg—X \quad (III)$$

in welcher

R und m die oben angegebene Bedeutung haben und

X für Halogen, insbesondere Chlor oder Brom steht,

in Gegenwart eines für eine Grignard-Reaktion üblichen Lösungsmittels, wie vorzugsweise Ether, bei Temperaturen zwischen etwa 30 bis etwa 80 °C umsetzt, oder

b) 1-Halogen-alkylalkohole der Formel

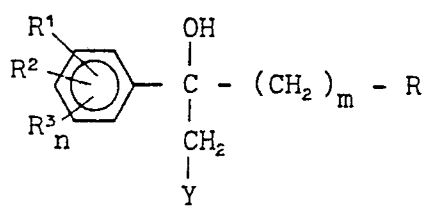

(IV)

in welcher

R, $R^1$, $R^2$, $R^3$, n und m die oben angegebene Bedeutung haben und

Y für Halogen, insbesondere Chlor oder Brom, steht,

mit Imidazol, gegebenenfalls in Gegenwart eines Säurebindemittels, wie z. B. einem Ueberschuß an Imidazol, und gegebenenfalls in Gegenwart eines inerten organischen Lösungsmittels, wie z. B. Acetonitril, bei Temperaturen zwischen 30 bis 200 °C, vorzugsweise bei der Siedetemperatur des Lösungsmittels, umsetzt. In manchen Fällen erweist es sich als vorteilhaft, das Imidazol in Form seiner Alkalisalze, wie dem Natrium- oder Kaliumsalz, einzusetzen.

Die erfindungsgemäß zu verwendenden Wirkstoffe, in denen der Index m für 0 steht, können auch

nach einem weiteren Verfahren erhalten werden, (vergleiche die Deutsche Offenlegungsschrift D 29 12 288), indem man

c) Oxirane der Formel

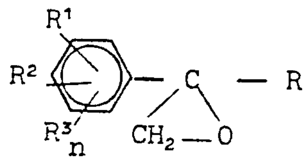

(V)

in welcher R, $R^1$, $R^2$, $R^3$ und n die oben angegebene Bedeutung haben,
mit Imidazol in Gegenwart eines Alkalialkoholats, wie z. B. Natriummethylat und in Gegenwart eines inerten organischen Lösungsmittels, wie z. B. Dimethylformamid, bei Temperaturen zwischen 30 und 100 °C umsetzt.

Die Imidazolylmethyl-phenyl-ketone der Formel (II) sind noch nicht bekannt. Sie lassen sich jedoch in allgemein üblicher und bekannter Weise herstellen, indem man entsprechende Phenacylhalogenide (das sind α-Halogen-acetophenon-Derivate) mit Imidazol in Gegenwart eines Verdünnungsmittels, wie beispielsweise Dimethylformamid, und in Gegenwart eines säurebindenden Mittels, wie insbesondere einem Überschuß an Imidazol, bei Temperaturen zwischen 20 und 80 °C umsetzt.

Die Grignard-Verbindungen der Formel (III) sind allgemein bekannte Verbindungen der organischen Chemie.

Die 1-Halogen-alkylalkohole der Formel (IV) sind noch nicht bekannt. Sie lassen sich jedoch in allgemein üblicher und bekannter Weise herstellen, indem man entsprechende Phenacylhalogenide mit Grignard-Verbindungen der Formel (III) nach Verfahrensvariante (a) umsetzt.

Die Oxirane der Formel (V) sind auch Gegenstand der erwähnten vorgängigen eigenen Anmeldung (vergleiche die Deutsche Offenlegungsschrift D 29 12 288) und können erhalten werden, indem man entsprechende Ketone entweder mit Dimethyloxosulfonium-methylid in an sich bekannter Weise in Gegenwart eines Verdünnungsmittels, wie beispielsweise Dimethylsulfoxid, bei Temperaturen zwischen 20 und 80 °C umsetzt ; oder mit Trimethylsulfoniummethylsulfat in an sich bekannter Weise in Gegenwart eines Zweiphasensystems und gegebenenfalls in Gegenwart eines Phasentransferkatalysators bei Temperaturen zwischen 0 und 100 °C umsetzt.

Zur Herstellung von Säureadditionssalzen der Verbindungen der Formel (I) kommen alle physiologisch verträglichen Säuren infrage, Hierzu gehören vorzugsweise die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure sowie Sulfonsäuren, wie z. B. p-Toluolsulfonsäure und 1,5-Naphthalindisulfonsäure.

Die Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolgt zur Bekämpfung von Podosphaera- u. Erysiphe-Arten eingesetzt werden, wie gegen den Erreger des echten Apfelmehltaus (Podosphaera leucotricha) und gegen den Erreger des echten Gurkenmehltaus (Erysiphe cichoracearum). Gute Erfolge werden auch bei der Bekämpfung von Getreidekrankheiten erzielt.

In bestimmten Aufwandmengen zeigen die erfindungsgemäßen Wirkstoffe auch eine wachstumsregulierende Wirkung.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe

mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid ; als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 % am Wirkungsort erforderlich.

In den nachfolgenden Beispielen werden die nachstehend angegebenen Verbindungen als Vergleichssubstanzen eingesetzt :

(A) = 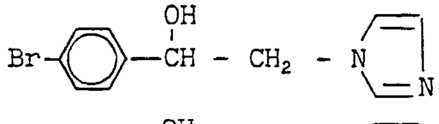

(B) = 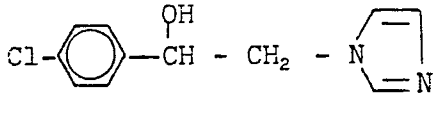

Beispiel A

Podosphaera-Test (Apfel)/protektiv

Lösungsmittel : 4,7 Gewichtsteile Aceton

Emulgator : 0,3 Gewichtsteile Alkylarylpolyglykolether
Wasser : 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20 °C und einer relativen Luftfeuchtigkeit von 70 % im Gewächshaus. Anschließend werden sie durch Bestäuben mit Konidien des Apfelmehltauerregers (Podosphaera leucotricha) inokuliert und in ein Gewächshaus mie einer Temperatur von 21 bis 23 °C und einer relativen Luftfeuchtigkeit von 70 % gebraucht.

10 Tage nach der Inokulation wird der Befall der Sämlinge bestimmt. Die erhaltenen Boniturwerte werden in Prozent Befall umgerechnet. 0 % bedeutet keinen Befall, 100 % bedeutet, daß die Pflanzen vollständig befallen sind.

In diesem Test zeigen z. B. folgende Verbindungen eine sehr gute Wirkung, die derjenigen der aus dem Stand der Technik bekannten Verbindung (A) deutlich überlegen ist :

Verbindungen gemäß Herstellungsbeispielen 5, 6 und 8.

## Beispiel B

### Erysiphe-Test (Gurken)/Protektiv

Lösungsmittel : 4,7 Gewichtsteile Aceton
Emulgator : 0,3 Gewichtsteile Alkyl-aryl-polyglykolether
Wasser : 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Gurkenpflanzen mit etwa drei Laubblättern bis zur Tropfnässe. Die Gurkenpflanzen verbleiben zur Trocknung 24 Stunden im Gewächshaus. Dann werden sie zur Inokulation mit Konidien des Pilzes Erysiphe cichoracearum bestäubt. Die Pflanzen werden anschließend bei 23 bis 24 °C und bei einer relativen Luftfeuchtigkeit von ca 75 % im Gewächshaus aufgestellt.

Nach 12 Stunden wird der Befall der Gurkenpflanzen bestimmt. Die erhaltenen Boniturwerte werden in Prozent Befall umgerechnet. 0 % bedeutet keinen Befall, 100 % bedeutet, daß die Pflanzen vollständig befallen sind.

Bei diesem Test zeigen z. B. die folgenden Verbindungen eine sehr gute Wirkung, die derjenigen der aus dem Stand der Technik bekannten Verbindung (B) deutlich überlegen ist :

Verbindung gemäß Herstellungsbeispiel 5.

## Beispiel C

### Myzelwachstums-Test

Verwendeter Nährboden :

20 Gewichtsteile Agar-Agar
200 Gewichtsteile Kartoffeldekokt
5 Gewichtsteile Malz
15 Gewichtsteile Dextrose
5 Gewichtsteile Pepton
2 Gewichtsteile Dinatriumhydrogenphosphat
0,3 Gewichtsteil Calciumnitrat

Verhältnis von Lösungsmittelgemisch zum Nährboden :

2 Gewichtsteile Lösungsmittelgemisch
100 Gewichtsteile Agarnährboden

Zusammensetzung Lösungsmittelgemisch :

0,19 Gewichtsteile Dimethylformamid oder Aceton
0,01 Gewichtsteile Emulgator Alkylarylpolyglykolether
1,80 Gewichtsteile Wasser
2 Gewichtsteile Lösungsmittelgemisch

Man vermischt die für die gewünschte Wirkstoffkonzentration im Nährboden nötige Wirkstoffmenge mit der angegebenen Menge des Lösungsmittelgemisches. Das Konzentrat wird im genannten Mengenverhältnis mit dem flüssigen, auf 42 °C abgekühlten Nährboden gründlich vermischt und in Petrischalen mit einem Durchmesser von 9 cm gegossen. Ferner werden Kontrollplatten ohne Präparatbeimischung aufgestellt.

Ist der Nährboden erkaltet und fest, werden die Platten mit den in der Tabelle angegebenen Pilzarten beimpft und bei etwa 21 °C inkubiert.

Die Auswertung erfolgt je nach Wachstumsgeschwindigkeit der Pilze nach 4-10 Tagen. Bei der Auswertung wird das radiale Myzelwachstum auf den behandelten Nährböden mit dem Wachstum auf dem Kontrollnährboden verglichen. Die Bonitierung des Pilzwachstums geschieht mit folgenden kennzahlen :

1 kein Pilzwachstum
bis 3 sehr starke Hemmung des Wachstums
bis 5 mittelstarke Hemmung des Wachstums
bis 7 schwache Hemmung des Wachstums
9 Wachstum gleich der unbehandelten Kontrolle

Bei diesem Test zeigen z. B. die folgenden Verbindungen eine sehr gute Wirkung, die derjenigen der aus dem Stand der Technik bekannten Verbindung (A) deutlich überlegen ist :

Verbindungen gemäß Herstellungsbeispielen 6, 14, 12, 5 und 9.

Folgende Pilze wurden zu diesem Versuch verwendet :

Fusarium nivale
Cochliobolus miyabeanus
Verticillium alboatrum
Pyricularia oryzae
Phialophora cinerescens
Helminthosporium gramineum
Mycosphaerella musicola
Pellicularia sasakii

## Herstellungsbeispiele

### Beispiel 1

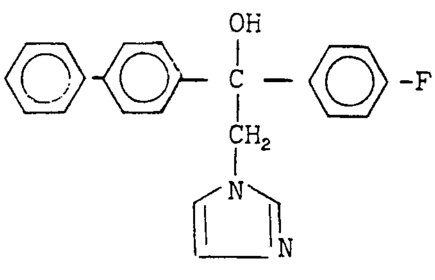

(Verfahren b)

Eine Lösung von 9,5 g (0,175 Mol) Natriummethylat in 49 ml Methylalkohol wird mit 20,2 g (0,297 Mol) Imidazol versetzt. Anschließend wird eine Lösung von 44,3 g (0,135 Mol) 1-(4-Biphenylyl)-2-chlor-1-(4-fluorphenyl)-ethanol in 103 ml Dimethylformamid zugetropft und 90 Minuten auf 60 °C erhitzt. Die Reaktionsmischung wird durch Abdestillieren der Lösungsmittel im Vakuum eingeengt und der Rückstand mit Wasser verrührt. Die verbleibenden Kristalle werden mit Acetonitril gewaschen und aus Ethylalkohol umkristallisiert. Man erhält 13,5 g (28 % der Theorie) 1-(4-Biphenylyl)-1-(4-fluorphenyl)-2-(imidazol-1-yl)-ethanol vom Schmelzpunkt 220 °C.

Herstellung des Ausgangsproduktes

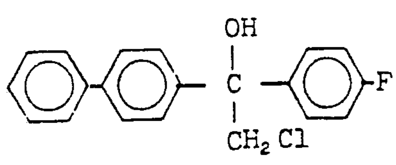

Zu einer Lösung von 4-Fluorphenyl-magnesiumbromid, erhalten aus 7,3 g (0,33 Mol) Magnesium und 52,5 g (0,3 Mol) 4-Fluorbrombenzol in 100 ml Diethylether, werden 34,5 g (0,15 Mol) 4-Phenylphenacylchlorid portionsweise zugegeben. Nach zweistündigem Erhitzen unter Rückfluß wird das Reaktionsgemisch auf wässrige Ammoniumchlorid-Lösung gegossen. Die abgetrennte Etherphase wird mit Wasser gewaschen, über Natriumsulfat getrocknet und eingedampft. Man erhält 44,3 g 1-(4-Biphenylyl)-2-chlor-1-(4-fluorphenyl)-ethanol.

Beispiel 2

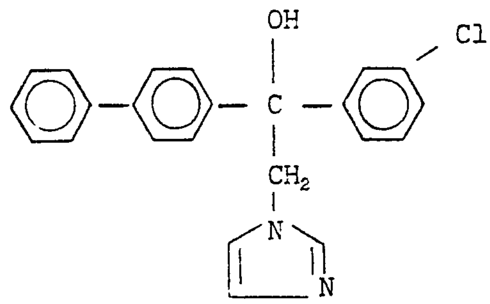

(Verfahren a)

Zu 21,6 g (0,1 Mol) 3-Chlorphenylmagnesiumbromid in 70 ml Ether (hergestellt aus 2,4 g (0,1 Mol) Magnesium und 19,1 g (0,1 Mol) 3-Bromchlorbenzol) werden portionsweise 13,1 g (0,05 Mol ) 4-Biphenylyl-(imidazol-1-yl-methyl)-keton gegeben. Nach Zugabe von 500 ml trockenem Toluol wird der Ether abdestilliert und die entstehende Suspension mit wässriger Ammoniumchloridlösung behandelt. Die Toluolphase wird abgetrennt, filtriert und über Natriumsulfat getrocknet. Man engt durch Abdestillieren des Toluols im Vakuum ein und verrührt den kristallinen Rückstand mit Acetonitril. Nach Umkristallisieren aus Ethanol erhält man 9,4 g (50 % der Theorie) 1-(4-Biphenylyl)-1-(3-chlorphenyl)-2-(imidazol-1-yl)-ethanol vom Schmelzpunkt 202 °C.

Beispiel 3

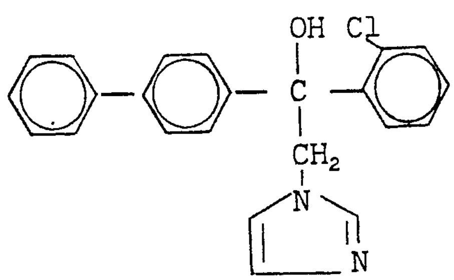

(Verfahren c)

3,5 g (0,065 Mol) Natriummethylat werden in 20 ml Methanol gelöst und mit 7,5 g (0,11 Mol) Imidazol versetzt. Dazu wird eine Lösung von 15 g (0,05 Mol) 2-(4-Biphenylyl)-2-(2-chlorphenyl)-oxiran in 75 ml Dimethylformamid getropft und das Reaktionsgemisch 1,5 Stunden auf 80 °C erhitzt. Danach wird das Reaktionsgemisch auf Wasser gegossen, die entstehenden Kristalle werden abfiltriert und getrocknet. Man erhält 14,7 g (79 % der Theorie) 1-(4-Biphenylyl)-1-(2-chlorphenyl)-2-imidazol-1-yl-ethanol vom Schmelzpunkt 222 °C.

Herstellung des Ausgangsproduktes

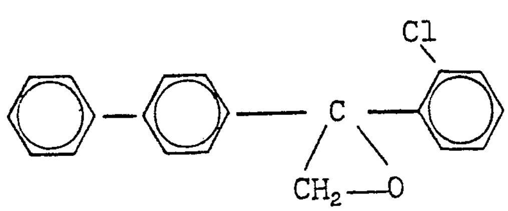

2,7 g (0,09 Mol) 80 %iges Natriumhydrid und 19,8 g (0,09 Mol) Trimethyloxosulfoniumjodid werden innerhalb 20 Minuten mit 90 ml Dimethylsulfoxid versetzt. Nach beendeter Wasserstoffentwicklung wird

eine Lösung von 22 g (0,075 Mol) 2-Chlor-4'-phenylbenzophenon in 60 ml Dimethylsulfoxid zugetropft und 1 Stunde bei 50 °C nachgerührt. Das erkaltete Reaktionsgemisch wird mit 200 ml Wasser versetzt und mit Ether ausgeschüttelt. Die Etherlösung wird mit Wasser gewaschen, über Natriumsulfat getrocknet und durch Abdestillieren des Lösungsmittels im Vakuum eingeengt. Der Rückstand wird aus Diisopropylether umkristallisiert. Man erhält 15 g (65 % der Theorie) 2-(4-Biphenylyl)-2-(2-chlorphenyl)-oxiran vom Schmelzpunkt 70 °C.

Beispiel 4

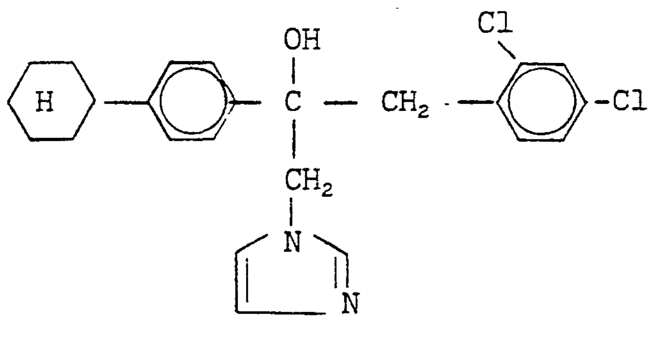

(Verfahren b)

Zu einer Suspension von 3,6 g (0,12 Mol) Natriumhydrid und 8,2 g (0,12 Mol) Imidazol in 200 ml Acetonitril wird eine Lösung von 39,8 g (0,1 Mol) 2-(4-Cyclohexylphenyl)-3-chlor-1-(2,4-dichlorphenyl)-propan-2-ol in 200 ml Acetonitril zugetropft. Nach dreistündigem Erhitzen auf 80 °C wird filtriert, die Kristallmasse mit Wasser gewaschen und aus Acetonitril umkristallisiert. Man erhält 24,9 g (58 % der Theorie) 2-(4-Cyclohexylphenyl)-1-(2,4-dichlorphenyl)-3-(imidazol-1-yl)-propan-2-ol vom Schmelzpunkt 142 °C.

Herstellung des Ausgangsproduktes

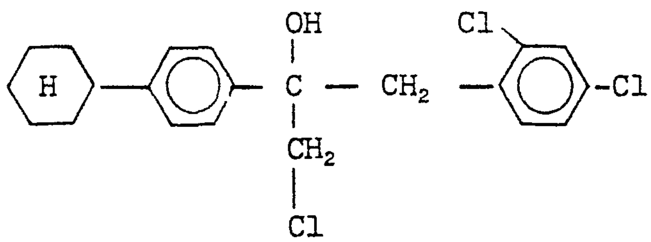

Zu einer Lösung von 0,4 Mol 2,4-Dichlorbenzylmagnesiumchlorid, erhalten aus 10,6 g (0,44 Mol) Magnesium und 70 g (0,4 Mol) 2,4-Dichlorbenzylchlorid in 150 ml Ether, wird eine Lösung von 47,3 g (0,2 Mol) 4-Cyclohexylphenacylchlorid in 200 ml Ether getropft. Das Reaktionsgemisch wird auf wässrige Ammoniumchlorid-Lösung gegossen, die Ether-Phase wird abgetrennt, mit Wasser gewaschen, über Natriumsulfat getrocknet und eingedampft. Man erhält 79,5 g (99 % der Theorie) 2-(4-Cyclohexyl)-3-chlor-1-(2,4-dichlorphenyl)-propan-2-ol vom Brechungsindex $n_D^{20}$ : 1,578 0.

In den Beispielen 1 bis 4 werden in entsprechender Weise folgende Verbindungen der allgemeinen Formel

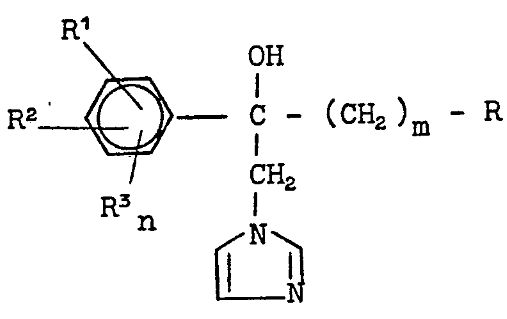

(I)

erhalten :

| Bsp. Nr. | R | $R^1$ | $R^2$ | $R^3_n$ | m | Schmelzpunkt (°C) |
|---|---|---|---|---|---|---|
| 5 | 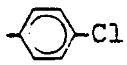 | 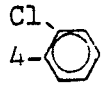 | H | - | 0 | 187 |
| 6 | 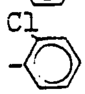 | 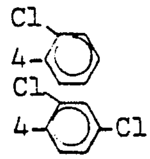 | H | - | 0 | 206 |
| 7 | 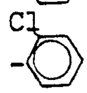 | | H | - | 0 | 220 |
| 8 | 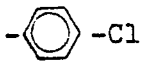 | 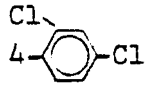 | H | - | 0 | 176 |
| 9 | 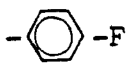 | 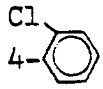 | H | - | 0 | 100 |
| 10 | 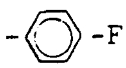 | 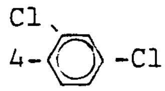 | H | - | 0 | 225 |
| 11 | 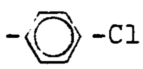 | 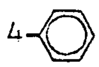 | H | - | 0 | 230 |
| 12 | 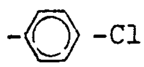 | 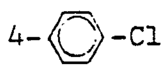 | H | - | 0 | 188 |
| 13 | 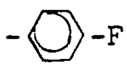 | 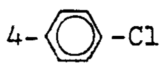 | H | - | 0 | 218 |
| 14 | 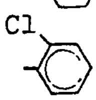 | 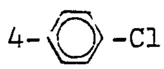 | H | - | 0 | 206 |
| 15 | 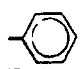 | 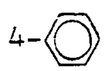 | H | - | 0 | 224 |
| 16 | 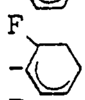 | 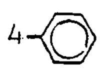 | H | - | 0 | 202 |
| 17 | 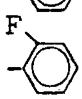 | 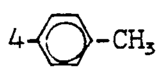 | H | - | 0 | 200 |
| 18 | 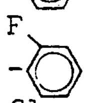 | 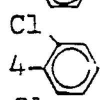 | H | - | 0 | 227 |
| 19 | 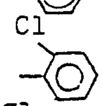 | 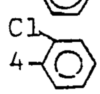 | H | - | 0 | 206 |
| 20 | 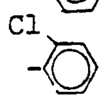 | 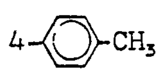 | H | - | 0 | 207 |
| 21 | 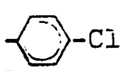 | 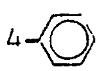 | H | - | 1 | 188 |
| 22 | 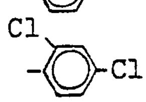 | 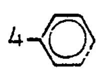 | H | - | 1 | 168 |
| 23 | 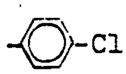 | 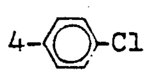 | H | - | 1 | 142 |
| 24 | 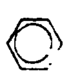 | 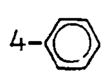 | H | - | 1 | 219 |

(Fortsetzung)

| Bsp. Nr. | R | $R^1$ | $R^2$ | $R^3_n$ | m | Schmelz-punkt(°C) |
|---|---|---|---|---|---|---|
| 25 | 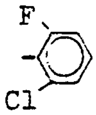 | 4- 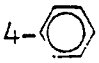 | H | - | 1 | 180 |
| 26 | 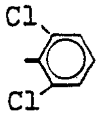 | 4- 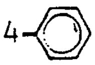 | H | - | 1 | 197 |
| 27 | 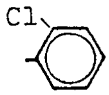 | 4- 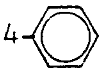 | H | - | 1 | 190 |
| 28 | 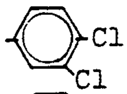 | 4- 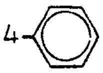 | H | - | 1 | 134 |
| 29 | 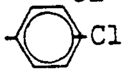 | $3,4-(CH_2)_3-$ | | - | 1 | 135 |
| 30 | 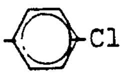 | 4- 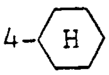 | H | - | 1 | 186 |
| 31 | 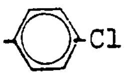 | $3,4-(CH_2)_3-$ | | - | 1 | 130 |
| 32 | 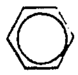 | 4- 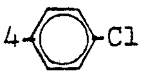 | H | - | 1 | 230 |
| 33 | 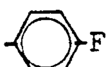 | 4- 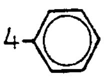 | H | - | 1 | 186 |
| 34 | 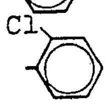 | 4- 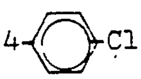 | H | - | 1 | 198 |
| 35 | 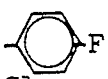 | 4- 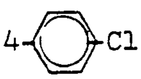 | H | - | 1 | 196 |
| 36 | 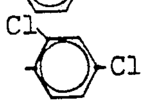 | 4- 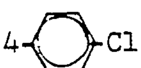 | H | - | 1 | 186 |
| 37 | 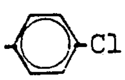 | 4- 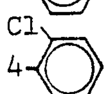 | H | - | 1 | 146 |
| 38 | 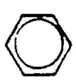 | 4- 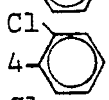 | H | - | 1 | 167 |
| 39 | 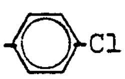 | 4- 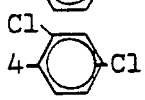 | H | - | 1 | 206 |
| 40 | 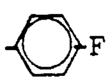 | 4- 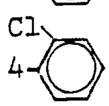 | H | - | 1 | 152 |
| 41 | 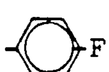 | 4- 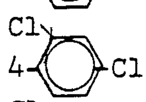 | H | - | 1 | 200 |
| 42 | 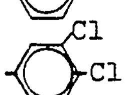 | 4- 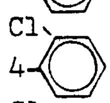 | H | - | 1 | 132 |
| 43 | 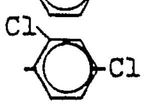 | 4- 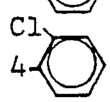 | H | - | 1 | 130 |

(Fortsetzung)

| Bsp. Nr. | R | $R^1$ | $R^2$ | $R^3_n$ | m | Schmelzpunkt (°C) |
|---|---|---|---|---|---|---|
| 44 | 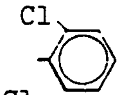 | 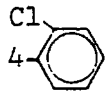 | H | - | 1 | 110 |
| 45 | 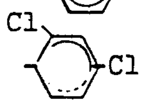 | 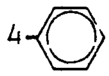 | H | - | 1 | 225 (xHCl) |
| 46 | 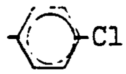 | 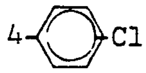 | H | - | 1 | 180 (xHCl) |
| 47 | 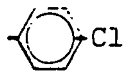 | 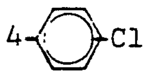 | H | - | 1 | 215 ($xH_2SO_4$) |
| 48 | 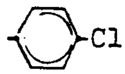 | 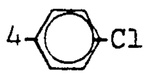 | H | - | 1 | 155 ($xHNO_3$) |
| 49 | 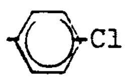 | 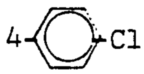 | H | - | 1 | 136 ($xCH_3COOH$) |
| 50 | 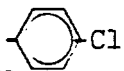 | 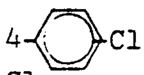 | H | - | 1 | 260 ($x H_3PO_4$) |
| 51 | 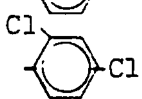 | 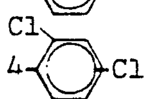 | H | - | 1 | 206 |
| 52 | 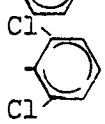 | 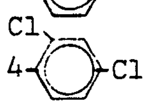 | H | - | 1 | 110 |
| 53 | 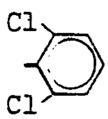 | 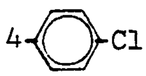 | H | - | 1 | 210 |
| 54 | 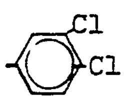 | 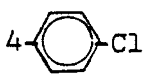 | H | - | 1 | 150 |

**Anspruch**

Verwendung von Hydroxyalkyl-imidazolen der allgemeinen Formel

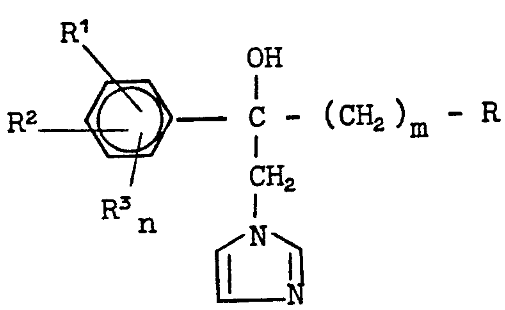

(I)

in welcher

R für gegebenenfalls durch Halogen oder Alkyl mit 1 bis 4 Kohlenstoffatomen substituiertes Phenyl, Naphthyl oder Tetrahydronaphthyl steht,

$R^1$ für gegebenenfalls durch Halogen, Alkyl oder Alkenyl mit jeweils bis zu 4 Kohlenstoffatomen substituiertes Phenyl oder Cycloalkyl mit 3 bis 7 Kohlenstoffatomen steht und

$R^2$ für Wasserstoff steht oder

$R^1$ und $R^2$ gemeinsam in o-Stellung zueinander für eine gegebenenfalls durch Halogen oder Alkyl mit bis zu 2 Kohlenstoffatomen substituierte Methylenbrücke mit 3 bis 5 Methylengruppen oder zusammen mit dem Phenylring für Naphthyl stehen,

$R^3$ für Halogen oder Alkyl mit 1 bis 4 Kohlenstoffatomen steht,

n für die Zahlen 0, 1 oder 2 steht und

m für die Zahlen 0 oder 1 steht,

oder deren physiologisch verträglichen Säureadditions-Salzen zur Bekämpfung von pflanzenschädigenden Pilzen.

**Claim**

Use of hydroxyalkyl-imidazoles of the general formula

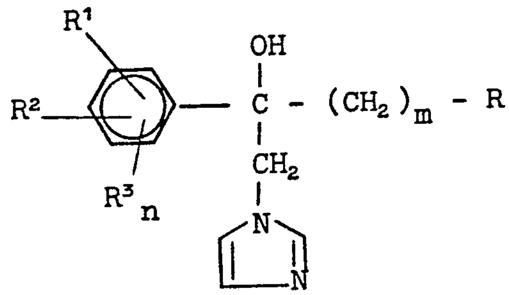

(I)

in which

R represents phenyl which is optionally substituted by halogen or alkyl with 1 to 4 carbon atoms, naphthyl or tetrahydronaphthyl,

$R^1$ represents phenyl or cycloalkyl with 3 to 7 carbon atoms, which are optionally substituted by halogen, alkyl or alkenyl with in each case up to 4 carbon atoms and

$R^2$ represents hydrogen or

$R^1$ and $R^2$ together, in an ortho-position relative to one another, represent a methylene bridge with 3 to 5 methylene groups which is optionally substituted by halogen or alkyl with up to 2 carbon atoms, or together with the phenyl ring represent naphthyl,

$R^3$ represents halogen or alkyl with 1 to 4 carbon atoms,

n represents the numbers 0, 1 or 2 and

m represents the numbers 0 or 1,

or physiologically acceptable acid addition salts thereof for combating plant-damaging fungi.

**Revendication**

Utilisation d'hydroxyalkyl-imidazoles de formule générale

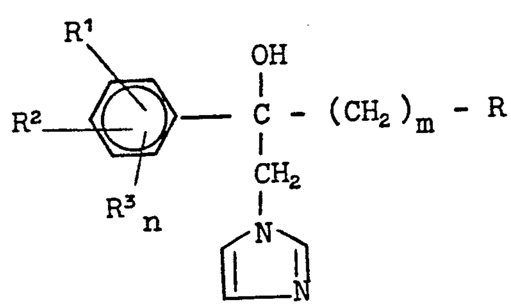

(I)

dans laquelle

R représente un groupe tétrahydronaphtyle, naphtyle ou phényle éventuellement substitué par un atome d'halogène ou par un groupe alkyle contenant 1 à 4 atomes de carbone,

$R^1$ représente un groupe cycloalkyle en $C_3$-$C_7$ ou un groupe phényle éventuellement substitué par un atome d'halogène, par un groupe alkyle ou par un groupe alcényle contenant chacun jusqu'à 4 atomes de carbone,

$R^2$ représente un atome d'hydrogène, ou

$R^1$ et $R^2$ ensemble en position o l'un par rapport à l'autre, représentent un pont méthylène comportant 3 à 5 groupes méthylène et éventuellement substitué par un atome d'halogène ou par un groupe alkyle contenant jusqu'à 2 atomes de carbone, ou encore, ensemble avec le noyau phényle, ils représentent un groupe naphtyle,

$R^3$ représente un atome d'halogène ou un groupe alkyle contenant 1 à 4 atomes de carbone,

n représente les nombres 0, 1 ou 2, et

m représente les nombres 0 ou 1,

ou de leurs sels d'addition d'acide physiologiquement compatibles pour combattre les champignons détériorant les plantes.